(19) Europäisches Patentamt
European Patent Office
Office européen des brevets

(11) Veröffentlichungsnummer: **0 000 704**
**A1**

(12) # EUROPÄISCHE PATENTANMELDUNG

(21) Anmeldenummer: 78100357.9

(51) Int. Cl.²: C 08 G 65/28, A 61 K 31/77

(22) Anmeldetag: 11.07.78

(30) Priorität: 21.07.77 DE 2732929

(43) Veröffentlichungstag der Anmeldung:
21.02.79 Patentblatt 79/4

(84) Benannte Vertragsstaaten:
BE CH DE FR GB LU NL SE

(71) Anmelder: Bayer Aktiengesellschaft
Zentralbereich Patente,Marken und Lizenzen Bayerwerk
D-5090 Leverkusen 1(DE)

(72) Erfinder: Steinert, Gerd, Dr.
Grundermühlenweg 8
D-5090 Leverkusen(DE)

(72) Erfinder: Horstmann, Harald, Dr.
Claudiusweg 19
D-5600 Wuppertal 1(DE)

(72) Erfinder: Sitt, Rüdiger, Dr.
Claudiusweg 9
D-5600 Wuppertal 1(DE)

(72) Erfinder: Krause, Hans Peter, Dr.
Wilkhausstrasse 107
D-5600 Wuppertal(DE)

(72) Erfinder: Plus, Walter, Dr.
In den Birken 75
D-5600 Wuppertal 1(DE)

(54) **Polyäther, Verfahren zu ihrer Herstellung sowie ihre Verwendung als Lipidabsorptionshemmer.**

(57) Verfahren zu ihrer Herstellung sowie ihre Verwendung als stoffwechselbeeinflussende Arzneimittel, insbesondere als Lipidabsorptionshemmer.

EP 0 000 704 A1

BAYER AKTIENGESELLSCHAFT
Zentralbereich
Patente, Marken und Lizenzen

5090 Leverkusen, Bayerwerk
KS/bc
Ib (Pha)

Polyäther, Verfahren zu ihrer Herstellung sowie ihre Verwendung als Lipidabsorptionshemmer

Vorliegende Erfindung betrifft neue Polyäther, mehrere Verfahren zu ihrer Herstellung sowie ihre Verwendung als stoffwechselbeeinflussende Arzneimittel, insbesondere als Lipidabsorptionshemmer.

Es ist bereits bekannt geworden, Polyäther, die aus Äthylenoxid- und Propylenoxidgruppierungen bestehen, herzustellen (vgl. US-Patent 2 674 619). Es ist ebenfalls bekannt, daß Polyoxialkylene mit Molgewichten von ca. 1000 bis ca. 11 000 oberflächenaktive Eigenschaften besitzen und darüberhinaus auch in pharmazeutischen Zubereitungen Verwendung finden können. In der US-Patentschrift 3 641 240 wird die Verwendung von Polyoxialkylenen mit einem Molekulargewicht von ca. 2000 bis 10 000 und einem Äthylenoxidgehalt von 50 bis 90 % als Antithrombotika beansprucht und beschrieben. In dieser Patentschrift wird ausdrücklich darauf hingewiesen, daß das anzuwendende

Polyoxialkylen mindestens 50 % Äthylenoxid enthalten soll.

In dem US-Patent 3 202 578 werden Polyoxialkylene beschrieben, die als Laxantia Verwendung finden können. In dieser Patentschrift wird neben der laxierenden Wirkung auch eine Wirkung auf die Cholesterinsenkung im Blut erwähnt. Nach den Ausführungen dieser Patentschrift sind solche Polyoxialkylene besonders als cholesterinsenkende Verbindungen geeignet, die ein Molekulargewicht von 7500 besitzen und 80 % Äthylenoxid enthalten.

Die vorliegende Erfindung betrifft neue Polyäther der allgemeinen Struktur

$$HO-(CH_2-CH_2-O)_X-(\overset{CH_3}{\overset{|}{C}}H-CH_2-O)_Y-(CH_2-CH_2-O)_Z-H \qquad (I)$$

in welcher

X, Y und Z so gewählt werden, daß ein Molekulargewicht von 3000 bis 5000 resultiert und daß der Propylenoxidanteil 60 bis 80 % und der Äthylenoxidanteil 20 bis 40 % beträgt.

Von besonderem Interesse sind Polyäther der allgemeinen Struktur (I) mit einem mittleren Molekulargewicht von 4000, einem Propylenoxidanteil von 70 % und einem Äthylenoxidanteil von 30 %.

Die Herstellung der erfindungsgemäßen Polyäther der allgemeinen Struktur (I) erfolgt, indem man

Propylenglykol in Gegenwart von Alkalihydroxid durch Polyaddition zunächst mit Propylenoxid und danach mit

einer entsprechenden Menge Äthylenoxid umsetzt, das Alkalihydroxid dann mit wäßriger Mineralsäure neutralisiert und nach Entwässerung des Polyäthers durch Salzfiltration entfernt.

Die erfindungsgemäßen Polyäther haben eine enge Molekulargewichtsverteilung.

Die Charakterisierung und Zusammensetzung der polymeren Oxialkylenverbindungen erfolgt analytisch durch Ermittlung des Molekulargewichts aus der Hydroxylzahl. Der Äthylenoxidgehalt wird aus dem $^1$H-NMR-Spektrum bestimmt.

Die erfindungsgemäßen Polyäther zeigen überraschenderweise sehr starke Wirkungen bei der Behandlung von Fett- und Kohlenhydratstoffwechselstörungen. Sie bewirken insbesondere eine Senkung des erhöhten Cholesterins im Serum und im Gewebe und vermindern gleichzeitig eine Hypertriglyceridämie. Die erfindungsgemäßen Verbindungen eignen sich zur Behandlung von Hyperlipoproteinämien, Atherosklerose, Adipositas und zur Behandlung hierdurch ausgelöster Stoffwechselstörungen.

Es ist als ausgesprochen überraschend zu bezeichnen, daß die erfindungsgemäßen Polyäther der vorliegenden Anmeldung ausgerechnet in diesem Molekulargewichtsbereich von 3 bis 5000, insbesondere 4000, und dem speziellen Propylenoxid/Äthylenoxid-Verhältnis eine so ausgeprägte hypolipidämische Wirkung besitzen. Da die erfindungsgemäßen Verbindungen neben dieser starken Wirkung gleichzeitig sehr gut verträglich sind, stellen sie eine Bereicherung der Pharmazie dar.

Zur vorliegenden Erfindung gehören pharmazeutische Zubereitungen, die neben nichttoxischen, inerten pharmazeutisch geeigneten Trägerstoffen eine oder mehrere Verbindungen der obigen Formel enthalten oder die aus einer oder mehreren Verbindungen der obigen Formel bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen, vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten genannt.

Tabletten, Dragees, Kapseln, Pillen und Granulate können den oder die Wirkstoffe neben den üblichen Trägerstoffen enthalten, wie (a) Füll- und Streckmittel, z.B. Stärken,

Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylakohol, Glycerinmonostearat, (h) Adsorptionsmittel, z.B. Kaolin und Bentonit und (i) Gleitmittel, z.B. Talkum-, Calcium- und Magnesiumstearat und feste Polyäthylenglykole oder Gemische der unter (a) - (i) aufgeführten Stoffe.

Die Tabletten, Dragees, Kapseln, Pillen und Granulate können mit den üblichen gegebenenfalls Opakisierungsmittel enthaltenden Ueberzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette z.B. Kakaofett und höhere Ester (z.B. $C_{14}$-Alkohol mit $C_{16}$-Fettsäure) oder Gemische dieser Stoffe.

Lösungen und Emulsionen können neben dem oder den Wirkstoffen die üblichen Trägerstoffe, wie Lösungsmittel, Lösungsvermittler und Emulgatoren, z.B. Wasser, Aethylalkohol, Isopropylalkohol, Aethylcarbonat, Aethylacetat, Benzylalkohol, Benzylbenzoat, Propylenglykol, 1,3-Butylenglykol, Dimethylformamid, Oele, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Aethylalkohol, Propylenglykol, Suspendiermittel z.B. äthoxylierte Isostearylalkohole, Polyoxyäthylensorbit- und -sorbitanester, mikrokristalline Cellulose, Aluminiummethanhydroxid, Bentonit, Agar-Agar und Traganth oder Gemische dieser Stoffe enthalten.

Die genannten Formulierungsformen können auch Färbemittel, Konservierungsstoffe sowie geruchs- und geschmachsverbessernde Zusätze, z.B. Pfefferminzöl und Eukalyptusöl, und Süßmittel, z.B. Saccharin, enthalten.

Die therapeutisch wirksamen Verbindungen sollen in den oben aufgeführten pharmazeutischen Zubereitungen in einer Konzentration von etwa 0,1 bis 99,5 , vorzugsweise von etwa 0,5 bis 95 Gewichtsprozent der Gesamtmischung, vorhanden sein.

Die oben aufgeführten pharmazeutischen Zubereitungen können außer Verbindungen der obigen Formel auch andere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der Verbindungen der obigen Formel sowie die Verwendung von pharmazeutischen Zubereitungen, die eine oder mehrere Verbindungen der oben angegebenen Formel enthalten, in der Human- und Veterinärmedizin zur Verhütung, Besserung und/oder Heilung der oben angeführten Erkrankungen.

Die Wirkstoffe oder die pharmazeutischen Zubereitungen können oral, parenteral, intraperitoneal und/oder rectal, vorzugsweise oral, appliziert werden.

Im allgemeinen hat es sich sowohl in der Human- als auch in der Veterinär-Medizin als vorteilhaft erwiesen, den oder die Wirkstoffe in Mengen von etwa 0,05 bis etwa 500, vorzugsweise 0,5 bis 200 mg/kg Körpergewicht je 24 Stdn., verteilt auf 1 bis 6 Verabreichungen, und zwar vor oder/und während oder/und nach der Mahlzeit zu applizieren. Eine Einzelgabe enthält den oder die Wirkstoffe vorzugsweise in Mengen von etwa 0,1 bis etwa 100 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der o.g. Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Beispiel 1

In einem 10 L-Glaskolben mit Heizbad, Rührer, Rückflußkühler, Epoxiddosiereinrichtung unter Stickstoff werden 93,5 g Propylenglykol und 62 g 50%ige Kalilauge vorgelegt. Durch azeotrope oder Vakuumdestillation wird entwässert. Danach werden bei 100-120° zunächst 4707 g Propylenoxid und danach 1200 g Aethylenoxid polyaddiert. Das Kaliumhydroxid wird durch Zugabe von verdünnter Schwefelsäure neutralisiert. Nach der Entwässerung des Polyäthers und destillativer Entfernung von leichtflüchtigen Anteilen wird das entstandene Kaliumsulfat durch Filtration entfernt. Das Molekulargewicht des Polyäthers, berechnet aus der OH-Zahl, beträgt 4000, und das Produkt enthält 20,0 Gew. % Aethylenoxid.

Beispiel 2

Durch Polyaddition von 4134,5 g (bzw. 3506,5 g) Propylenoxid und 1800,0 g (bzw. 2400,0 g) Äthylenoxid an 93,5 g Propylenglykol in Gegenwart von 62,0 g 50%iger Kalilauge wird nach dem in Beispiel 1 beschriebenen Verfahren ein Polyäther mit 30 (bzw. 40) Gew.-% Äthylenoxid und einem Molgewicht von 4000 erhalten.

Patentansprüche

1) Polymere Oxialkylene der allgemeinen Struktur

$$HO-(CH_2-CH_2-O)_X-(\overset{CH_3}{\overset{|}{C}H}-CH_2-O)_Y-(CH_2-CH_2-O)_Z-H$$

in welcher

X, Y und Z so gewählt sind, daß ein Molekulargewicht von 3000 bis 5000 resultiert, der Propylenoxidanteil 60 bis 80 % und der Äthylenoxidanteil 20 bis 40 % beträgt.

2) Polymere Polyalkylene gemäß Anspruch 1, dadurch gekennzeichnet, daß X, Y und Z so gewählt sind, daß ein Molekulargewicht von 4000 resultiert.

3) Polymere Oxialkylene gemäß Anspruch 1, dadurch gekennzeichnet, daß der Propylenoxidanteil 70 % und der Äthylenoxidanteil 30 % beträgt.

4) Polymere Oxialkylene gemäß Anspruch 1 mit einem Molekulargewicht von 4000, einem Propylenoxidanteil von 70 % und einem Äthylenoxidanteil von 30 %.

5) Verfahren zur Herstellung von polymeren Oxialkylenen gemäß Formel (I) in Anspruch 1, dadurch gekennzeichnet, daß man Propylenglykol in Gegenwart eines Alkalihydroxids zunächst mit Propylenoxid und danach mit einer entsprechenden Menge Äthylenoxid umsetzt und das Alkalihydroxid nach Neutralisation mit Mineralsäure durch Salzfiltration entfernt.

6) Arzneimittel mit hypolipidämischer Wirkung, dadurch gekennzeichnet, daß es ein polymeres Oxiäthylen gemäß Anspruch 1 enthält.

7) Verwendung von polymerem Oxiäthylen gemäß Anspruch 1 zur Behandlung von Fett- und Kohlenhydratstoffwechselstörungen.

0000761

Europäisches Patentamt

# EUROPÄISCHER RECHERCHENBERICHT

Nummer der Anmeldung

EP 78 10 0357

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| | NL - A - 66 18108 (SMITH KLINE & FRENCH LABORATORIES)<br>* Seite 1, Zeilen 1-4; Patentansprüche * | 1-7 | C 08 G 65/28<br>A 61 K 31/77 |
| | FR - M - 1 116 M (B.A.F.MIRANDE u.a.)<br>* Seite 1, linke Spalte; Zusammenfassung * | 1-7 | |
| | GB - A - 610 505 (HAROLD EDWIN POTTS)<br>* Patentansprüche * | 1-5 | **RECHERCHIERTE SACHGEBIETE (Int. Cl.²)**<br>C 08 G 65/26<br>C 08 G 65/28<br>A 61 K 31/77 |

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 09-10-1978 | LENSEN |

EPA form 1503.1 06.78